# EUROPEAN PATENT SPECIFICATION

(11) **EP 3 971 726 B1**
(45) Date of publication and mention of the grant of the patent: **29.11.2023**
(21) Application number: 21189864.8
(22) Date of filing: 05.08.2021
(51) Int. Cl.: A61M 5/14, A61M 5/168

(54) **OUTPUT DEVICE, OUTPUT METHOD, AND RECORDING MEDIUM**
AUSGABEVORRICHTUNG, AUSGABEVERFAHREN UND AUFZEICHNUNGSMEDIUM
DISPOSITIF DE SORTIE, PROCÉDÉ DE SORTIE ET SUPPORT D'ENREGISTREMENT

(30) Priority: 18.09.2020 JP 2020157727; 18.09.2020 JP 2020157808
(43) Date of publication of application: 23.03.2022
(73) Proprietor: Casio Computer Co., Ltd., Tokyo 151-8543 (JP)
(72) Inventor: Yoshizawa, Hiroaki, Tokyo 205-8555 (JP)
(74) Representative: Grünecker Patent- und Rechtsanwälte PartG mbB

(56) References cited:
- JP-A- 2004 236 911
- JP-U- 3 207 941
- US-A- 4 709 331

## Description

### FIELD

Embodiments described herein relate generally to an output device, an output method, and a recording medium.

### BACKGROUND

Nurses perform dedicated calculations in many cases. For example, Japanese Registered Utility Model No. 3103399 discloses a nurse calculator having a function of executing calculations frequently performed by a nurse to support work of nurses.

JP 3207941 U relates to a drip rate adjustment aid, which displays drip rates of an infusion based on a total quantity of liquid and a target time for the total infusion. An operator can select different predetermined time intervals. JP 2004236911 A relates to a drip auxiliary meter for storing information related to an infusion such as the kind, total amount, time, speed or the like of an infusion. The drip rate may be output by light or sound signal for a desired time-interval.

Calculations frequently performed by nurses include calculation of drip infusion speed. An example of the calculation of drip infusion speed is calculation of converting the number of drops per minute with a drip infusion apparatus into the number of drops per 10 seconds or 15 seconds.

To convert the number of drops per minute into the number of drops per 10 seconds, the number of drops per minute is divided by 6. To convert the number of drops per minute into the number of drops per 15 seconds, the number of drops per minute is divided by 4. However, there is the problem that the result of conversion of the number of drops per 10 seconds or 15 seconds is not always an integer.

For this reason, it is required to round off the converted number of drops into the nearest integer to round the error. Whether to adjust the number into the number of drops per 10 seconds or the number of drops per 15 seconds should be determined in consideration of the time to the finish of the drip infusion to select the one having the smaller whole error.

### BRIEF SUMMARY

The invention is defined by the features of the independent claims, with preferred embodiments being specified in the dependent claims.

### BRIEF DESCRIPTION OF THE DRAWINGS

The components in the drawings are not necessarily to scale relative to each other.
FIG. 1 is a front view illustrating an external appearance structure of an electronic apparatus including an output device to which an output method according to a first embodiment is applied.
FIG. 2 is a block diagram illustrating configuration of an electronic circuit of the output device included in the electronic apparatus.
FIG. 3 is a table illustrating calculation examples executed with the output device.
FIG. 4 is a schematic diagram illustrating a display example displayed on a display of the output device.
FIG. 5 is a flowchart illustrating an example of flow of drop number calculation display processing executed with the output device.
FIG. 6 is a flowchart illustrating an example of flow of drop number calculation execution processing executed with the output device.
FIG. 7 is a block diagram illustrating configuration of an electronic circuit of an output device included in an electronic apparatus according to a second embodiment.
FIG. 8 is a flowchart illustrating an example of flow of drop number calculation display processing executed with the output device.

### DETAILED DESCRIPTION

Embodiments of the present invention will now be explained hereinafter with reference to drawings.

### (First Embodiment)

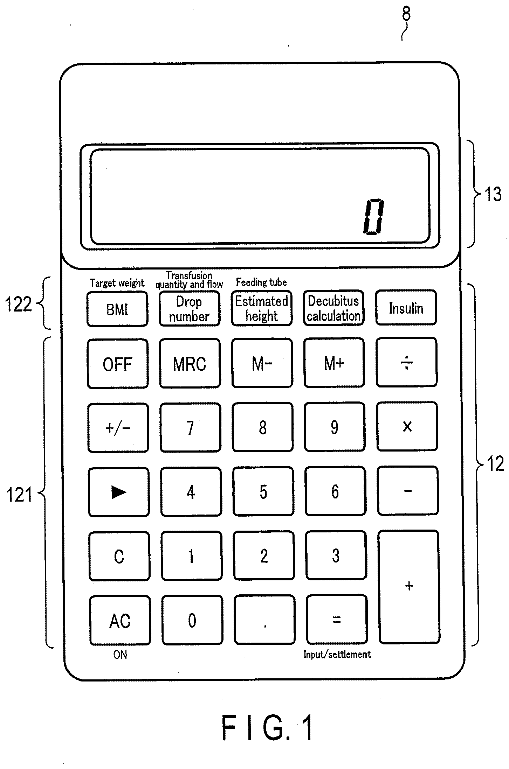

FIG. 1 is a front view illustrating an external appearance structure of an electronic apparatus including an output device to which an output method according to a first embodiment is applied.

FIG. 1 illustrates an electronic apparatus 8 configured as a nurse calculator, as an example. However, the electronic apparatus 8 is not limited to the case of being configured as a nurse calculator, but may be configured as a tablet terminal, a smartphone, a mobile phone, a touchpanel PDA (personal digital assistants), an electronic book, and/or a portable came console, or the like.

An electronic apparatus (not illustrated) not provided with physical keys (buttons) as provided in a nurse calculator, such as a tablet terminal, displays a software keyboard similar to keys, and processing is executed in response to key operations on the software keyboard.

The electronic apparatus 8 configured as a nurse calculator is formed in a small size enabling the user to sufficiently hold the calculator with one hand and operate the calculator with one hand, for the necessity of portability thereof. A key input unit 12 and a display 13 are provided on a front surface of a main member of the electronic apparatus 8.

The key input unit 12 includes a numerical value/arithmetic operation symbolkey group 121 to instruct calculation and/or execution of programs, and a function key group 122 to start various dedicated functions.

Keys arranged as the numerical value/arithmetic operation symbolkey group 121 are [0] to [9] (numerical value) keys, [+], [-], [×], and [÷] (four arithmetic operations) keys, a [=] (execution) key, a [+/-] (plus and minus signs) key, a [C] (clear) key, an [AC] (all clear) key, and [MRC], [M-], {M+} (memory operation) keys and the like. The [AC] (all clear) key also functions as a power button.

Keys arranged as the function key group 122 are a [BMI] key to calculate BMI, a [drop number] key to calculate the number of drops of drip infusion, an [estimated height] key to calculate the estimated height, a [decubitus] key to execute calculation relating to decubitus, and an [insulin] key to calculate the insulin transfusion quantity and the like.

The display 13 is formed of a liquid crystal display unit of a dot-matrix type. When the electronic apparatus 8 is a tablet terminal, the display 13 is formed of a liquid crystal display unit provided by superimposing a touch panel.

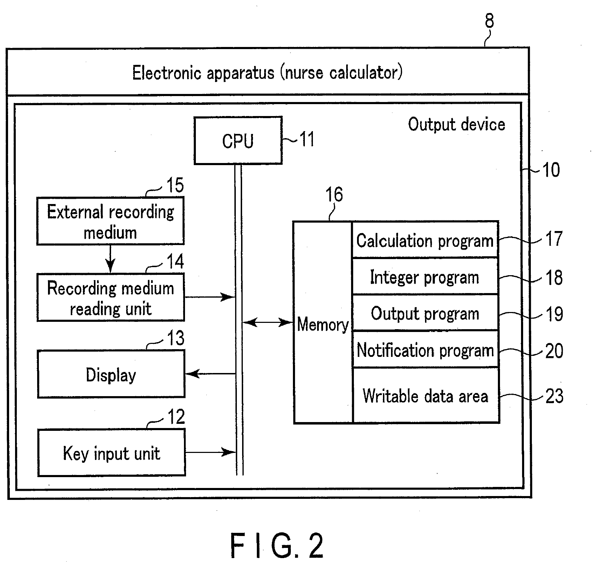

FIG. 2 is a block diagram illustrating configuration of an electronic circuit of the output device included in the electronic apparatus.

An output device 10 to which the output method according to the first embodiment is applied is started when the [drop number] key of the electronic apparatus 8 is pressed.

The electronic circuit of the output device 10 includes a CPU 11 being a computer, a memory 16, and a recording medium reading unit 14, in addition to the key input unit 12 and the display 13 described above. These elements are used in common also when the electronic apparatus 8 operates as a device other than the output device 10, such as a device executing an ordinary calculator function, BMI calculation, estimated height calculation, decubitus calculation, and insulin calculation. The following explanation illustrates the structure for operating as the output device 10.

The memory 16 stores therein a calculation program 17, an integer program 18, an output program 19, and a notification program 20.

The CPU 11 controls operations of each of the units of the circuit in accordance with the programs 17 to 20, and executes various arithmetic operations relating to the number of drops of drip infusion using information input from the key input unit 12.

The programs 17 to 20 may be stored in the memory 16 in advance, or read from an external recording medium 15, such as a memory card, into the memory 16 via the recording medium reading unit 14 and stored therein.

The programs 17 to 20 are unrewritable by the user's operation of the key input unit 12.

A writable data area 23 is secured in the memory 16, as an area storing information rewritable by the user, in addition to information unrewritable by the user. The writable data area 23 is an area successively receiving key code information input with the key input unit 12 by key operations, and storing results of arithmetic operations executed for the key code information with the programs 17 to 20.

In the output device 10 configured as described above, the CPU 11 controls operations of the units of the circuit in accordance with commands described in the programs 17 to 20. The output device 10 operates as explained hereinafter by cooperation of software with hardware.

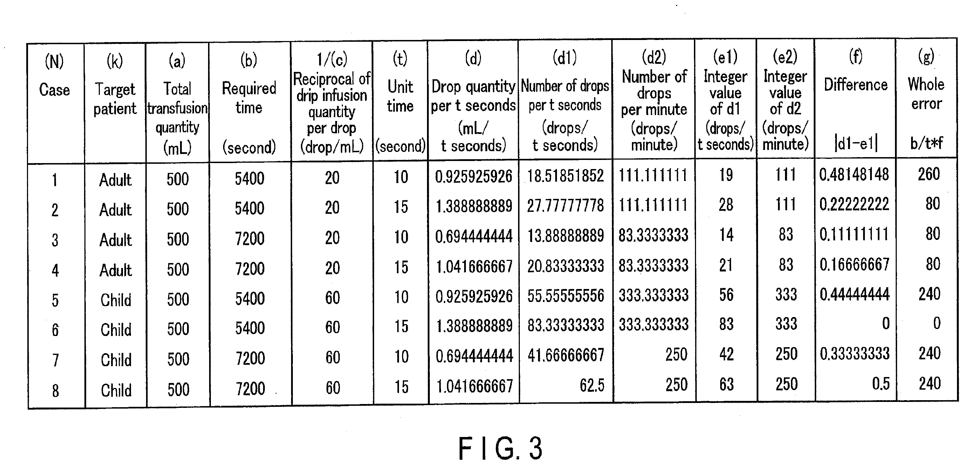

FIG. 3 is a table illustrating calculation examples executed with the output device 10.

The calculation program 17 executes display on the display 13 to inquire a target patient (adult or child) k of the transfusion set, a total transfusion quantity a to be infused, and required time b to be spent for drip infusion. On the basis of the target patient k, the total transfusion quantity a, and the required time b input by the nurse in response to the display, a drip number d1 per each of a plurality of unit times t (for example, 10 seconds and 15 seconds) is calculated. A drip quantity c per drop is 1 (mL) for 20 (drops) when the target patient k is an adult, and 1 (mL) for 60 (drops) when the target patient k is a child. The calculation program 17 also calculates a drop number d2 per unit time T (for example, 1 minute) longer than the unit times t (for example, 10 seconds and 15 seconds).

Each of Cases 1 and 2 in FIG. 3 is the case in which the target patient k is an adult, that is, the reciprocal of the drip quantity c per drop is 20 (drop/mL), the total transfusion quantity a is 500 (mL), and the required time b is 1.5 (hours), that is, 5400 (seconds). The unit time t is 10 (seconds) in Case 1, and 15 (seconds) in Case 2.

In this state, the drop number d2 per unit time T (1 minute) is, 111.11111... (drops) in each of Cases 1 and 2. The drop number d1 per unit time t is 18.518518... (drops) in Case 1, and 27.777778... (drops) in Case 2.

The integer program 18 executes fraction processing for each of the drop numbers d1 calculated for the respective unit times t, and changes the drop numbers d1 into integers to acquire integer valuea e1. The integer program 18 also executes fraction processing for the drop numbers d2 for the respective unit times T in the same manner, and changes the drop numbers d2 into integers to acquire integer values e2. The fraction processing may be, for example, rounding, rounding up or down the fraction after the decimal point, and rounding down the digit not exceeding 5 and rounding up the digit exceeding 5, but not limited thereto.

When rounding is executed as the fraction processing, in the example of FIG. 3, 19 (drops) is acquired as the integer number e1 from 18.518518... being the drop number d1 in Case 1, and 28 (drops) is acquired as the integer number e1 from 27.777777... being the drop number d1 in Case 2. Both in Cases 1 and 2, 111 (drops) is acquired as the integer value e2 from 111.111111... (drops) being the drop number d2 per unit time T (1 minute).

The output program 19 preferentially outputs the drop number per unit time having the smaller whole error g per required time b generated with a rounding error caused by fraction processing, in the integer values e1 of Case 1 and Case 2 changed into integers with the integer program 18. For this reason, first, the output program 19 determines a difference f between the drop number d1 calculated per unit time t and the corresponding integer value e1 for each of Case 1 and Case 2.

When the explanation is continued with reference to the example of FIG. 3, in Case 1, a difference f between 18.518518... (drops) being the drop number d1 and 19 (drops) srving as the corresponding integer e1 is 0.481481... (drops). By contrast, in Case 2, a difference f between 27.777777... (drops) being the drop number d1 and 28 (drops) srving as the corresponding integer e1 is 0.222222... (drops).

Thereafter, the output program 19 multiplies a value acquired by dividing the required time b by the corresponding unit time t by the corresponding difference f for each of the unit times t, to acquire the whole error g being the product thereof.

When the explanation is continued with reference to the example of FIG. 3, in Case 1, when a value acquired by dividing 5400 (seconds) being the required time b by 10 (seconds) being the corresponding unit time t is multiplied by 0.481481... (drops) being the corresponding difference f, the whole error g being the product is 260 (drops). By contrast, in Case 2, when a value acquired by dividing 5400 (seconds) being the required time b by 15 (seconds) being the corresponding unit time t is multiplied by 0.222222... (drops) being the corresponding difference f, the whole error g being the product is 80 (drops).

The output program 19 also preferentially outputs the drop number per unit time t having the smaller whole error g in the two unit times t, and displays the drop number on the display 13. The output program 19 also outputs the unit time T and the integer number e2, and displays them on the display 13.

When the explanation is continued with reference to the example of FIG. 3, because the whole error g is 260 (drops) in Case 1 and the whole error g is 80 (drops) in Case 2, the output program 19 preferentially outputs the value "28 (drops)" being the drop number per 15 seconds being the unit time t of Case 2 having the smaller whole error g, and displays the value, for example, "28 (drops/15 seconds)", on the display 13.

In addition, when the integer value e2 is 111 (drops) in the unit time T (1 minute), the output program 19 outputs the value "1 minute" being the unit time T and the value "111 (drops)" being the integer value e2, and displays the value, for example, "111 (drops/minute)" on the display 13.

The notification program 20 notifies the user of the drop timing determined on the basis of the drop number per unit time t output from the output program 19 by, for example, blinking the display of the LCD being the display 13 .

When the explanation is continued with reference to the example of FIG. 3, when the drop number output from the output program 19 is 28 (drops) in 15 seconds of Case 2, the drop timing occurs 28 times in 15 seconds. Specifically, because "15/28 = 0.535714... (seconds)" is satisfied, the drop timing occurs every 0.535714... seconds. The notification program 20 blinks the display of the LCD being the display 13 at each of the drop timings.

The following is an explanation of an operation example of the output device 10 configured as described above according to the first embodiment.

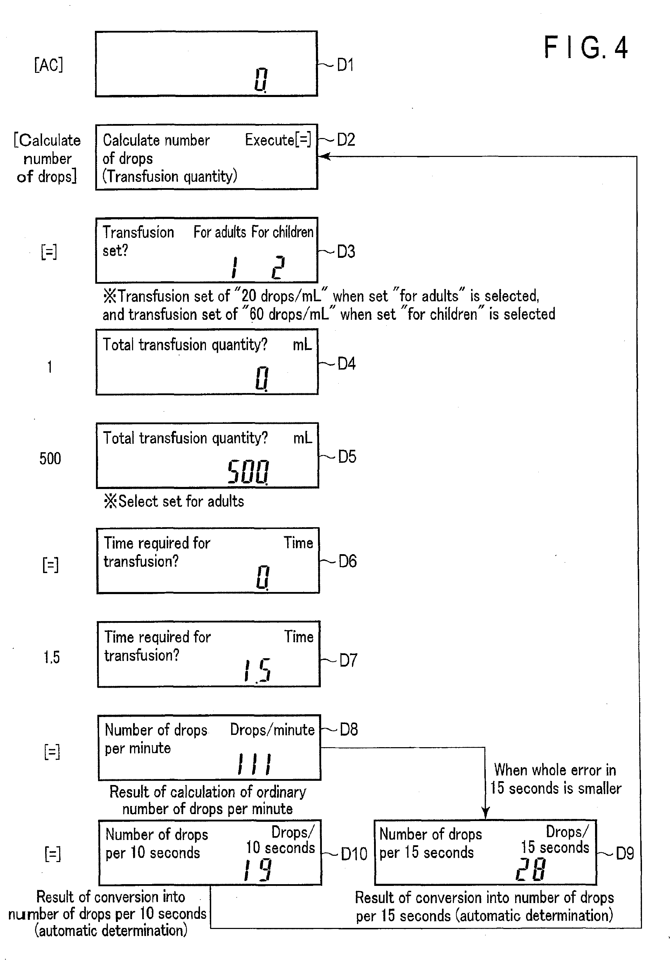

FIG. 4 is a schematic diagram illustrating a display example displayed on the display of the output device.

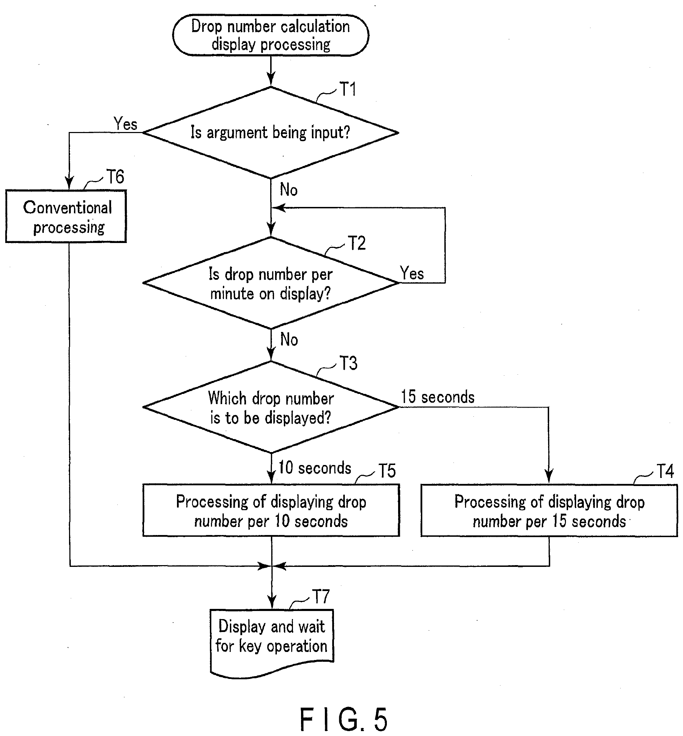

FIG. 5 is a flowchart illustrating an example of flow of drop number calculation display processing executed with the output device.

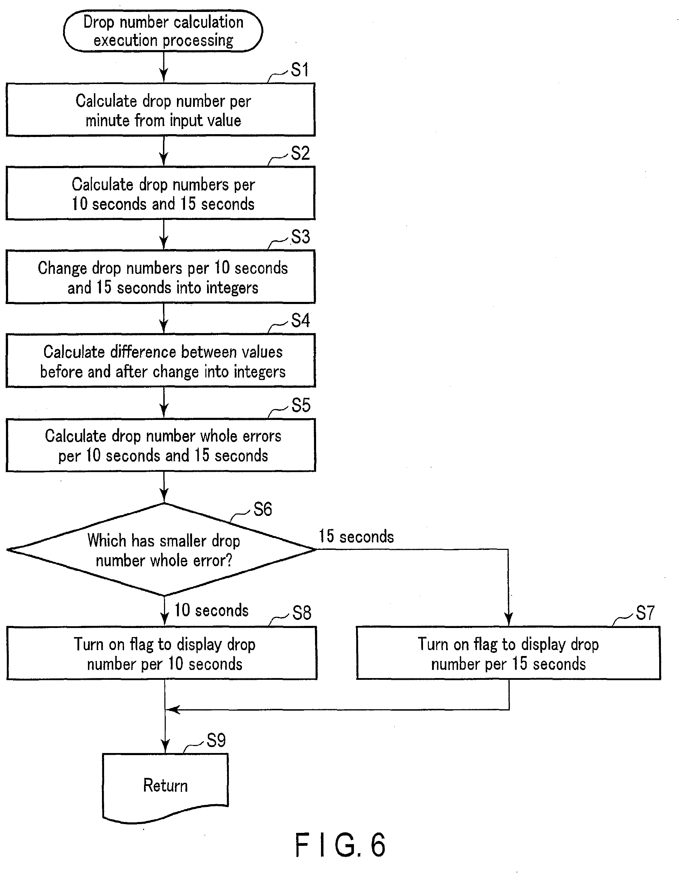

FIG. 6 is a flowchart illustrating an example of flow of drop number calculation execution processing executed with the output device.

When the [AC] (all clear) key is pressed (D1 in FIG. 4), the electronic apparatus 8 is powered. Thereafter, when the [drop number] key is pressed, the electronic apparatus 8 starts operation as the output device 10 to calculate the drop number (D2 in FIG. 4) of drip infusion and display the calculation results.

The display 13 performs display to promote input of information required for calculation of the drop number. First, the display 13 performs display to inquire whether the transfusion set of the target patient k is a transfusion set for adults or children (D3 in FIG. 4). In response to the display, when the nurse inputs the value "1" using the key input unit 12, the transfusion set is recognized as a set for "adult". When the nurse inputs the value "2", the transfusion set is recognized as a set for "child". Suppose that the value "1" is input in the example illustrated in FIG. 4. In this manner, the calculation program 17 recognizes that the target patient k is an adult, and the reciprocal of the drip quantity per drop is set as "20 (drops/mL)".

Thereafter, the display 13 performs display to inquire the total transfusion quantity a in the unit of "mL" (D5 in FIG. 4). In response to the display, when the nurse inputs the value "500" using the key input unit 12, the total transfusion quantity a is set as "500 (mL)".

Thereafter, the display 13 performs display to inquire the required time b to be spent for drip infusion in the unit of "hour" (D6 in FIG. 4). In response to the display, when the nurse inputs the value "1.5" using the key input unit 12 (D7 in FIG. 4), the required time b to be spent for drip infusion is recognized as "1.5 (hours)", and set as "5400 (seconds)".

The state up to this step corresponds to the process "steps T1→T6→T7→T1" in FIG. 5.

On the basis of the input conditions described above, the calculation program 17 calculates the drop number d2 per unit time T (1 minute). In the case of the input conditions described above, the calculation program 17 acquires the result "111.111111... (drops)" as the drop number d2. The drop number d2 is changed into the integer number e2 by fraction processing executed with the integre program 18. Specifically, bcuase the drop number d2 is 111.111111... (drops), the result "111 (drops)" is acquired as the integer number e2, and displayed on the display 13 with the output program 19 (D8 in FIG. 4, T2 in FIG. 5, and S1 in FIG. 6).

In addition, the calculation program 17 calculates the drop numer d1 per unit time t for each of Cases 1 and 2 on the basis of the input conditions described above (S2 in FIG. 6). In the case of the input conditions described above, the result "18.518518... (drops)" is acquired as the drop number d1 for Case 1, and the result "27.777777... (drops)" is acquired as the drop number d1 for Case 2.

Each of the drop numbers d1 acquired at Step S2 is changed into the integer number e1 by the fraction processing executed with the integer program 18 (S3 in FIG. 6). The value "19 (drops)" is acquired as the integer value e1 for the drop number d1 per 10 seconds in Case 1, and the value "28 (drops)" is acquired as the integer value e1 for the drop number d1 per 15 seconds in Case 2.

Thereafter, the output program 19 determines a difference f between the drop number d1 acquired for each unit time t and the corresponding integer value e1 (S4 in FIG. 6). In Case 1, a difference f between 18.518518... (drops) being the drop number d1 and 19 (drops) being the corresponding integer value e1 is 0.481481... (drops). In Case 2, a difference f between 27.777777... (drops) being the drop number d1 and 28 (drops) being the corresponding integer value e1 is 0.222222... (drops).

In addition, the output program 19 calculates the whole error g per required time b for each of the unit times t (S5 in FIG. 6). The whole error g is acquired as the product acquired by multiplying a value acquired by dividing the required time b to be spent for for drip infusion by the unit time t by the corresponding difference f. As illustrated in FIG. 3, the whole error g is 260 (drops) in Case 1, while the whole error g is 80 (drops) in Case 2.

Thereafter, the output program 19 preferentially outputs the integer e1 having the smaller whole error g in the integer values e1 acquired for the two unit times t (that is, 10 (seconds) in Case 1 and 15 (seconds) for Case 2), and the integer value e1 is displayed on the display 13 (S6 in FIG. 6 and T3 in FIG. 5).

The whole error g is 260 (drops) in Case 1, and the whole error g is 80 (drops) in Case 2. Accordingly, the output program 19 outputs the integer value e1 "28 (drops)" being the drop number per unit time t of Case 2 having the smaller whole error g, and the integer value is displayed as, for example, "28 (drops/15 seconds)" on the display 13 as illustrated in D9 of FIG. 4 (T3 (15 seconds) and T4 in FIG. 5, S6 (15 seconds) and S7 in FIG. 6).

If the whole error g of Case 1 is smaller than that of Case 2, the integer value e1 "19 (drops)" being the drop number per 10 seconds being the unit time t of Case 1 is outputted and displayed as, for example, "19 (drops/10 seconds)" on the display 13 as illustrated in D10 of FIG. 4 (T3 (10 seconds) and T5 in FIG. 5, S6 (10 seconds) and S8 in FIG. 6).

Thereafter, on the basis of the drop number being the integer value e1 output from the output program 19 and the corresponding unit time t, the notification program 20 notifies the user of the drop timing by, for example, blinking display of the LCD being the display 13 (T7 in FIG. 5).

As explained above, the output device and the electronic apparatus including the output device according to the first embodiment calculate the drop number in each of a plurality of unit times to adjust the drop number of drip infusion, and preferentially output the drop number per unit time t having the smaller whole error g per required time b. This structure enables the nurse to adjust the drip infusion number with high accuracy to satisfy the designated drip infusion conditions.

### (Second Embodiment)

The second embodiment relates to an electronic apparatus including an output device with a more simplified structure than that of the first embodiment.

The first embodiment has been explained using Cases 1 and 2 illustrated in FIG. 3. The first embodiment illustrates the example of preferentially displaying the integer value e1 "28 (drops)" being the drop number per 15 seconds being the unit time t of Case 2 having the smaller whole error g, because the whole error g of Case 1 having the unit time t of 10 seconds is 260 (drops) and the whole error g of Case 2 having the unit time t of 15 seconds is 80 (drops).

However, because both Case 1 and Case 2 have the drop quantity of 111 (drops) per 1 minute, the difference "180 (drops)" being the difference in whole error g corresponds to a time difference "1.621621... (minutes)". In Cases 1 and 2, because the required time b is 1.5 hour, that is, 90 minutes, the difference "1.621621... (minutes)" is only an error "1.801801... (%)".

For this reason, in the case where the transfusion set is capable of permitting an error to a certain degree, the output program 19 is not required to calculate the whole error g, and it is possible to provide an electronic apparatus including an output device with a more simplified structure than that of the first embodiment.

The present embodiment illustrates an electronic apparatus including such an output device.

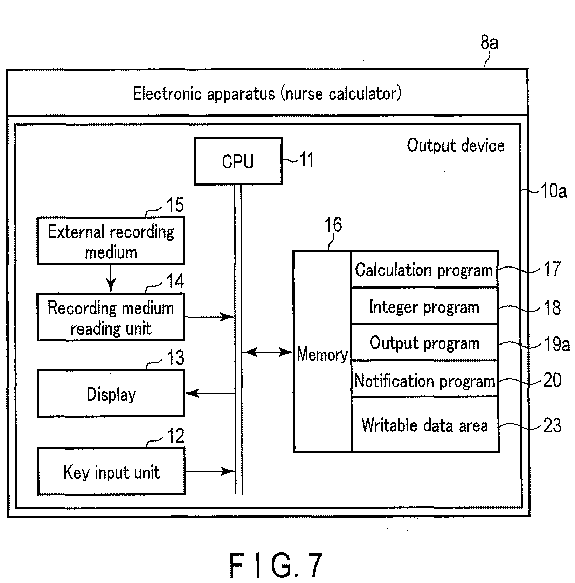

FIG. 7 is a block diagram illustrating configuration of an electronic circuit of an output device included in an electronic apparatus according to a second embodiment.

The block diagram illustrated in FIG. 7 has a structure including an output program 19a with a more simplified function than that of the output program 19, instead of the output program 19 in the block diagram illustrated in FIG. 2. The structures and operations of the calculation program 17, the integer program 18, and the notification program 20 are described in the first embodiment, and an overlapping explanation herein is omitted.

The output program 19a outputs the unit time T and the integer value e2, and displays them on the display 13. Although this structure is similar to the output program 19 explained in the first embodiment, the output program 19a preferentially outputs the drop number per unit time t having the smaller round error acquired by fraction processing executed with the integer program 18 in the two unit times t, unlike the first embodiment.

The latter operation of the output program 19a will now be explained using the example of Cases 1 and 2 illustrated in FIG. 3. Because the difference (f) is 0.481481... (drops) when the unit time t is 10 (seconds) as in Case 1 and the difference (f) is 0.222222... (drops) when the unit time t is 15 (secods) as in Case 2, the output program 19a outputs the integer value e1 "19 (drops)" being the drop number per 15 seconds being the unit time t of Case 2 having the smaller difference f in Cases 1 and 2, and displays the integer value e1 on the display 13.

The notification program 20 notifies the user of the drop number output from the output program 19a and the drop timing determined from the corresponding unit time t, by, for example, blinking the display of the LCD being the display 13. As described above, when the integer value e1 "19 (drops)" being the drop number per 15 seconds being the unit time t is output from the output program 19, the drop timing occurs 28 times in 15 seconds. Specifically, because "15/28 = 0.535714... (seconds)" is satisfied, the drop timing occurs every 0.535714... (seconds). The notification program 20 blinks the display of the LCD being the display 13 at each of the drop timings.

The following is an explanation of an operation example of the output device 10a configured as described above according to the second embodiment.

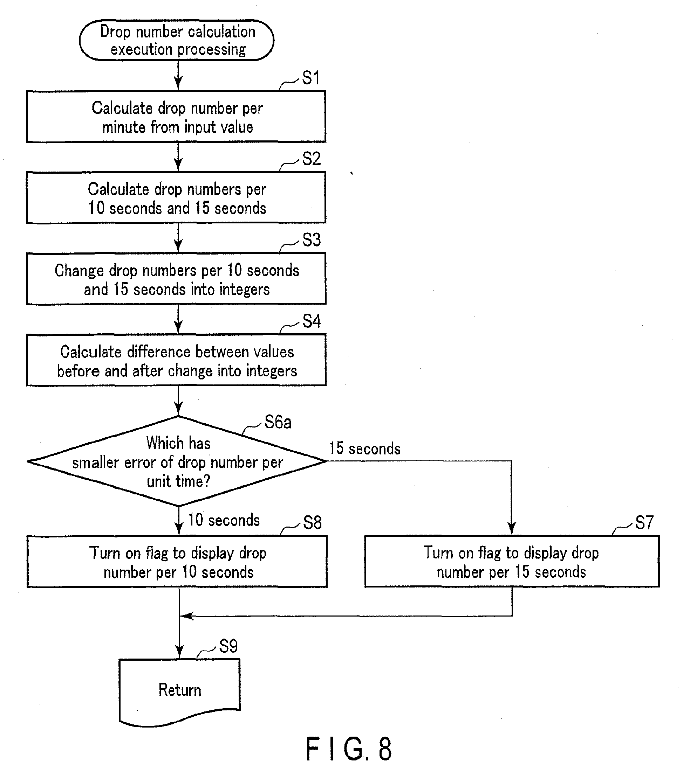

FIG. 8 is a flowchart illustrating an example of flow of drop number calculation display processing executed with the output device.

The flow of drop number calculation display processing is similar to those of FIG. 4 and FIG. 6. In addition, the input conditions used in the following explanation are Cases 1 and 2 illustrated in FIG. 3, in the same manner as the first embodiment.

The flowchart illustrated in FIG. 8 is acquired by removing Step S5 from the flowchart illustrated in FIG. 6, and replacing Step S6 by Step S6a. For this reason, the following explanation only simply illustrates the steps other than Step S6a.

When the [AC] (all clear) key is pressed (D1 in FIG. 4), the electronic apparatus 8a is powered. Thereafter, when the [drop number] key is pressed, the electronic apparatus 8a starts operation as the output device 10 to calculate the drop number (D2 in FIG. 4) of drip infusion and display the calculation results.

At Step S1, in the same manner as the first embodiment, the calculation program 17 and the integer program 18 acquire the result "111 (drops)" as the integer value e2 of the drop number d2 per unit time T (1 minute), and the output program 19a displays the result on the display 13 (D8 in FIG. 4 and T2 in FIG. 5).

At Step S2, the calculation program 17 calculates the value "18.518518... (drops)" as the drop number d1 per 10 seconds being the unit time t for Case 1, and the value "27.777777... (drops)" as the drop number d1 for Case 2.

At Step S3, the integer program 18 executes fraction processing for the drop numbers d1 for the respective unit times t, and the respective integer numbers e1 are acquired. In this manner, the value "19 (drops)" is acquired as the integer value e1 of the drop number d1 in Case 1, and the value "28 (drops)" is acquired as the integer value e1 of the drop number d1 in Case 2.

At Step S4, the output program 19a determines a difference f between the drop number d1 and the corresponding integer number e1 for each of the cases. In this manner, the value "0.481481... (drops)" is acquired as the difference f in Case 1, and the value "0.222222... (drops)" is acquired as the difference f in Case 2.

At Step S6a, the output program 19a selects one of Cases 1 and 2 having the smaller difference f, and outputs and displays the integer value e1 as the drop number per unit time t of the selected case on the display 13.

Because the difference f is 0.481481... (drops) in Case 1 and the difference f is 0.222222... (drops) in Case 2, the output program 19 selects Case 2, the integer value e1 "28 (drops)" is output from the output program as the drop number per 15 seconds being the unit time t of Case 2, and displayed, for example, as "28 (drops/15 seconds)" on the display 13.

In this case, on the basis of the value "15 seconds" being the unit time t of Case 2 and the value "28 (drops)" being the integer value e1, the notification program 20 determines the drop timings corresponding to "15/28 = 0.535714... (seconds)". The user is notified of the drop timings by, for example, blinking the display of the LCD being the display 13 in accordance with the timings.

As described above, the output device and the electronic apparatus including the output device according to the second embodiment achieve a more simplified structure than that of the first embodiment, when they are used for calculation of the drop number of a transfusion set capable of permitting an error to a certain degree.

The output device and the electronic apparatus including the output device as described above are also capable of outputting the unit time and the drop number having a small influence of an error, and the nurse is enabled to adjust the drip infusion number with high accuracy to satisfy the designated drip infusion conditions.

Specifically, the output device according to the embodiments described above calculates a first operation execution number and a second operation execution number, on the basis of a total quantity of drip infusion (target) output by a plurality of executions of a drop operation (certain operation) with a drip infusion device (external device), required time to be spent for output of the target of the total quantity with the external device, and a quantity of the target output per one execution of the certain operation, the first operation execution number serves as an execution number of the certain operations to be executed per first unit time, and the second operation execution number serves as an execution number of the certain operations to be executed per second unit time different from the first unit time. The output device executes fraction processing for each of the calculated first operation execution number and the calculated second operation execution number to change them into integers, and preferentially outputs the operation execution number per unit time of one of the first operation execution number and the second operation execution number having been changed into integers, on the basis of results of the fraction processing. This structure enables the nurse to adjust the drip infusion number with high accuracy to satisfy the designated drip infusion conditions.

Although the embodiments described above illustrate the examples in which the present invention is applied to drop number calculation, but the present invention is not limited to application to drop number calculation. For example, the present invention is applicable to calculation in the case where adjustment of a quantity of medical powder per bag is required in powder dividing and packing processing executed with an external device within a predetermined time.

Specifically, the structure calculates a first operation execution number and a second operation execution number, on the basis of a total quantity of medical powder output by a plurality of executions of a powder dividing and packing operation (dividing) with an external device (total quantity of target output by a plurality of executions of a certain operation), required time to be spent for output of the medical powder of the total quantity with the external device (required time to be spent for output of the target of the total quantity with the external device), and a quantity of the medical powder output per one execution of the powder dividing and packing operation (quantity of the target output per one execution of the certain operation), the first operation execution number serves as an execution number of the powder dividing and packing operations to be executed per first unit time, and the second operation execution number serves as an execution number of the powder dividing and packing operations to be executed per second unit time different from the first unit time. The output device executes fraction processing for each of the calculated first operation execution number and the calculated second operation execution number to change them into integers, and preferentially outputs the operation execution number per unit time of one of the first operation execution number and the second operation execution number having been changed into integers, on the basis of results of the fraction processing.

In the operation, the operation execution number having a smaller round error acquired with the fraction processing may be preferentially outputted in the first operation execution number and the second operation execution number having been changed into integers, or the operation execution number having a smaller whole error per the required unit time generated with a round error acquired with the fraction processing may be preferentially outputted in the first operation execution number and the second operation execution number having been changed into integers.

The invention of the present application is not limited to the embodiments, but may be variously modifieid when it is carried out within the range not departing from the gist of the invention. The embodiments include inventions of various stages, and various inventions can be extracted by proper combinations of a plurality of disclosed constituent elements. For example, even when some constituent elements are deleted from the constituent elements disclosed in each embodiment or some of the constituent elements are combined in different forms, the structures acquired by deletion or combination of the constituent elements may be extracted as inventions, when the structures can solve the problem described in the section "Problem to be Solved by Invention" and produce the effects described in the section "Effects of Invention".

## Claims

1. An output device (10) comprising at least one processor (11) executing a program stored in a storage unit, the processor (11) being configured to:
calculate (S2) a first operation execution number and a second operation execution number, on the basis of a total quantity of a target output by a plurality of executions of a certain operation executed by an external device, required time to be spent for output of the target of the total quantity executed by the external device, and a quantity of the target output per one execution of the certain operation, the first operation execution number being an execution number of the certain operations to be executed per first unit time, the second operation execution number being an execution number of the certain operations to be executed per second unit time different from the first unit time;
**characterized in that** the processor is further configured to: execute (S3) fraction processing for each of the calculated first operation execution number and the calculated second operation execution number to change them into integers; and
output the operation execution number per unit time of one of the first operation execution number and the second operation execution number having been changed into integers, wherein
the one of the first operation execution number and the second operation execution number is an operation execution number having a smaller round error (S6a) acquired with the fraction processing, or
the one of the first operation execution number and the second operation execution number is an operation execution number having a smaller whole error (S6) per the required unit time generated with a round error acquired with the fraction processing.

2. The output device (10) according to claim 1, **characterized in that**
the total quantity of the target output by a plurality of executions of the certain operation executed by the external device is a total transfusion quantity of liquid of drip infusion to be dropped,
the required time to be spent for output of the target of the total quantity executed by the external device is required time to be spent for dropping of the liquid of the drip infusion, the quantity of the target output per one execution of the certain operation is a liquid quantity of the drip infusion per drop,
the first operation execution number is a first drop number, and
the second operation execution number is a second drop number.

3. The output device (10) according to claim 2, **characterized in that** the processor (11) notifies drop timings determined on the basis of the output drop number per unit time.

4. The output device (10) according to claim 3, **characterized in that** the processor (11) notifies the drop timings by blinking display of the output drop number per unit time at the drop timings.

5. The output device (10) according to any one of claims 1 to 4, **characterized in that** the second unit time is longer than the first unit time.

6. An output method for an output device (10), the method comprises the steps of:
calculating (S2) a first operation execution number and a second operation execution number, on the basis of a total quantity of a target output by a plurality of executions of a certain operation executed by an external device, required time to be spent for output of the target of the total quantity executed by the external device, and a quantity of the target output per one execution of the certain operation, the first operation execution number being an execution number of the certain operations to be executed per first unit time, the second operation execution number being an execution number of the certain operations to be executed per second unit time different from the first unit time;
said method being **characterized in that** it further comprises the steps of:
executing (S3) fraction processing for each of the calculated first operation execution number and the calculated second operation execution number to change them into integers; and
outputting the operation execution number per unit time of one of the first operation execution number and the second operation execution number having been changed into integers, wherein
the one of the first operation execution number and the second operation execution number is an operation execution number having a smaller round error (S6a) acquired with the fraction processing, or
the one of the first operation execution number and the second operation execution number is an operation execution number having a smaller whole error (S6) per the required unit time generated with a round error acquired with the fraction processing.

7. The output method according to claim 6, **characterized in that**
the total quantity of the target output by a plurality of executions of the certain operation executed by the external device is a total transfusion quantity of liquid of drip infusion to be dropped,
the required time to be spent for output of the target of the total quantity executed by the external device is required time to be spent for dropping of the liquid of the drip infusion, the quantity of the target output per one execution of the certain operation is a liquid quantity of the drip infusion per drop,
the first operation execution number is a first drop number, and
the second operation execution number is a second drop number.

8. A program which when executed with at least one processor (11) of the output device (10) of claim 1 performs the method of claim 6.

9. The program according to claim 8, **characterized in that**
the total quantity of the target output by a plurality of executions of the certain operation executed by the external device is a total transfusion quantity of liquid of drip infusion to be dropped,
the required time to be spent for output of the target of the total quantity executed by the external device is required time to be spent for dropping of the liquid of the drip infusion,
the quantity of the target output per one execution of the certain operation is a liquid quantity of the drip infusion per drop,
the first operation execution number is a first drop number, and
the second operation execution number is a second drop number.

## Patentansprüche

1. Ausgabevorrichtung (10), die wenigstens einen Prozessor (11) umfasst, der ein in einer Speicher-Einheit gespeichertes Programm ausführt, wobei der Prozessor (11) konfiguriert ist zum:
Berechnen (S2) einer ersten Anzahl von Ausführungen eines Vorgangs und einer zweiten Anzahl von Ausführungen eines Vorgangs auf Basis einer Gesamtmenge für ein Objekt, die mittels einer Vielzahl von Ausführungen eines von einer externen Vorrichtung ausgeführten bestimmten Vorgangs ausgegeben wird, erforderlicher Zeit, die für Ausgabe der Gesamtmenge für das Objekt aufzuwenden ist, die von der externen Vorrichtung ausgeführt wird, sowie einer für das Objekt ausgegebenen Menge pro einer Ausführung des bestimmten Vorgangs, wobei die erste Anzahl von Ausführungen eines Vorgangs eine pro erste Zeiteinheit auszuführende Anzahl von Ausführungen der bestimmten Vorgänge ist, die zweite Anzahl von Ausführungen eine pro zweite Zeiteinheit, die sich von der ersten Zeiteinheit unterscheidet, auszuführende Anzahl von Ausführungen der bestimmten Vorgänge ist;
**dadurch gekennzeichnet, dass** der Prozessor des Weiteren konfiguriert ist zum:
Ausführen (S3) von Bruch-Verarbeitung jeweils für die berechnete erste Anzahl von Ausführungen eines Vorgangs sowie die berechnete zweite Anzahl von Ausführungen eines Vorgangs zum Umwandeln derselben in ganze Zahlen; und
Ausgeben der Anzahl von Ausführungen eines Vorgangs pro Zeiteinheit der ersten Anzahl von Ausführungen eines Vorgangs oder der zweiten Anzahl von Ausführungen eines Vorgangs, die in ganze Zahlen umgewandelt worden sind, wobei
die erste Anzahl von Ausführungen eines Vorgangs oder die zweite Anzahl von Ausführungen eines Vorgangs eine Anzahl von Ausführungen eines Vorgangs ist, die einen geringeren bei der Bruch-Verarbeitung entstandenen Rundungsfehler (S6a) aufweist, oder
die erste Anzahl von Ausführungen eines Vorgangs oder die zweite Anzahl von Ausführungen eines Vorgangs eine Anzahl von Ausführungen eines Vorgangs ist, die einen geringeren Gesamtfehler (S6) pro der benötigten Zeiteinheit aufweist, der mit einem bei der Bruch-Verarbeitung entstandenen Rundungsfehler erzeugt wird.

2. Ausgabevorrichtung (10) nach Anspruch 1, **dadurch gekennzeichnet, dass** die Gesamtmenge für das Objekt, die mittels einer Vielzahl von Ausführungen des von der externen Vorrichtung ausgeführten bestimmten Vorgangs ausgegeben wird, eine Gesamt-Transfusionsmenge von bei Tropfinfusion zu verabreichender Flüssigkeit ist,
die erforderliche Zeit, die für Ausgabe der Gesamtmenge für das Objekt aufzuwenden ist, die von der externen Vorrichtung ausgeführt wird, erforderliche Zeit ist, die zum Verabreichen der Flüssigkeit bei der Tropfinfusion aufzuwenden ist,
die für das Objekt ausgegebene Menge pro eine Ausführung des bestimmten Vorgangs eine Flüssigkeitsmenge der Tropfinfusion pro Tropfen ist,
die erste Anzahl von Ausführungen eines Vorgangs eine erste Tropfen-Anzahl ist, und die zweite Anzahl von Ausführungen eines Vorgangs eine zweite Tropfen-Anzahl ist.

3. Ausgabevorrichtung (10) nach Anspruch 2, **dadurch gekennzeichnet, dass** der Prozessor (11) über auf der Basis der ausgegebenen Tropfen-Anzahl pro Zeiteinheit bestimmte Tropf-Zeitpunkte informiert.

4. Ausgabevorrichtung (10) nach Anspruch 3, **dadurch gekennzeichnet, dass** der Prozessor (11) über die Tropf-Zeitpunkte mittels Blinkanzeige der zu den Tropf-Zeitpunkten ausgegebenen Tropfen-Anzahl pro Zeiteinheit informiert.

5. Ausgabevorrichtung (10) nach einem der Ansprüche 1 bis 4, **dadurch gekennzeichnet, dass** die zweite Zeiteinheit länger ist als die erste Zeiteinheit.

6. Ausgabeverfahren für eine Ausgabevorrichtung (10), wobei das Verfahren die folgenden Schritte umfasst:
Berechnen (S2) einer ersten Anzahl von Ausführungen eines Vorgangs und einer zweiten Anzahl von Ausführungen eines Vorgangs auf Basis einer Gesamtmenge für ein Objekt, die mittels einer Vielzahl von Ausführungen eines von einer externen Vorrichtung ausgeführten bestimmten Vorgangs ausgegeben wird, erforderlicher Zeit, die für Ausgabe der Gesamtmenge für das Objekt aufzuwenden ist, die von der externen Vorrichtung ausgeführt wird, sowie einer für das Objekt ausgegebenen Menge pro einer Ausführung des bestimmten Vorgangs, wobei die erste Anzahl von Ausführungen eines Vorgangs eine pro erste Zeiteinheit auszuführende Anzahl von Ausführungen der bestimmten Vorgänge ist, die zweite Anzahl von Ausführungen eine pro zweite Zeiteinheit, die sich von der ersten Zeiteinheit unterscheidet, auszuführende Anzahl von Ausführungen der bestimmten Vorgänge ist;
wobei das Verfahren **dadurch gekennzeichnet ist, dass** es des Weiteren die folgenden Schritte umfasst:
Ausführen (S3) von Bruch-Verarbeitung jeweils für die berechnete erste Anzahl von Ausführungen eines Vorgangs sowie die berechnete zweite Anzahl von Ausführungen eines Vorgangs zum Umwandeln derselben in ganze Zahlen; und
Ausgeben der Anzahl von Ausführungen eines Vorgangs pro Zeiteinheit der ersten Anzahl von Ausführungen eines Vorgangs oder der zweiten Anzahl von Ausführungen eines Vorgangs, die in ganze Zahlen umgewandelt worden sind, wobei die erste Anzahl von Ausführungen eines Vorgangs oder die zweite Anzahl von Ausführungen eines Vorgangs eine Anzahl von Ausführungen eines Vorgangs ist, die einen geringeren bei der Bruch-Verarbeitung entstandenen Rundungsfehler (S6a) aufweist, oder
die erste Anzahl von Ausführungen eines Vorgangs oder die zweite Anzahl von Ausführungen eines Vorgangs eine Anzahl von Ausführungen eines Vorgangs ist, die einen geringeren Gesamtfehler (S6) pro der benötigten Zeiteinheit aufweist, der mit einem bei der Bruch-Verarbeitung entstandenen Rundungsfehler erzeugt wird.

7. Ausgabeverfahren nach Anspruch 6, **dadurch gekennzeichnet, dass** die Gesamtmenge für das Objekt, die mittels einer Vielzahl von Ausführungen des von der externen Vorrichtung ausgeführten bestimmten Vorgangs ausgegeben wird, eine Gesamt-Transfusionsmenge von bei Tropfinfusion zu verabreichender Flüssigkeit ist,
die erforderliche Zeit, die für Ausgabe der Gesamtmenge für das Objekt aufzuwenden ist, die von der externen Vorrichtung ausgeführt wird, erforderliche Zeit ist, die zum Verabreichen der Flüssigkeit bei der Tropfinfusion aufzuwenden ist,
die für das Objekt ausgegebene Menge pro eine Ausführung des bestimmten Vorgangs eine Flüssigkeitsmenge der Tropfinfusion pro Tropfen ist,
die erste Anzahl von Ausführungen eines Vorgangs eine erste Tropfen-Anzahl ist, und die zweite Anzahl von Ausführungen eines Vorgangs eine zweite Tropfen-Anzahl ist.

8. Programm, das, wenn es mit wenigstens einem Prozessor (11) der Ausgabevorrichtung (10) nach Anspruch 1 ausgeführt wird, das Verfahren nach Anspruch 6 durchführt.

9. Programm nach Anspruch 8, **dadurch gekennzeichnet, dass** die Gesamtmenge für das Objekt, die mittels einer Vielzahl von Ausführungen des von der externen Vorrichtung ausgeführten bestimmten Vorgangs ausgegeben wird, eine Gesamt-Transfusionsmenge von bei Tropfinfusion zu verabreichender Flüssigkeit ist,
die erforderliche Zeit, die für Ausgabe der Gesamtmenge für das Objekt aufzuwenden ist, die von der externen Vorrichtung ausgeführt wird, erforderliche Zeit ist, die zum Verabreichen der Flüssigkeit bei der Tropfinfusion aufzuwenden ist,
die für das Objekt ausgegebene Menge pro eine Ausführung des bestimmten Vorgangs eine Flüssigkeitsmenge der Tropfinfusion pro Tropfen ist,
die erste Anzahl von Ausführungen eines Vorgangs eine erste Tropfen-Anzahl ist, und die zweite Anzahl von Ausführungen eines Vorgangs eine zweite Tropfen-Anzahl ist.

## Revendications

1. Dispositif de sortie (10) comprenant au moins un processeur (11) exécutant un programme stocké dans une unité de stockage, le processeur (11) étant conçu pour :
calculer (S2) un premier nombre d'exécution d'opérations et un second nombre d'exécution d'opérations, sur la base d'une quantité totale d'une sortie cible par une pluralité d'exécutions d'une certaine opération exécutée par un dispositif externe, d'un temps requis à passer pour une sortie de la cible de la quantité totale exécutée par le dispositif externe, et d'une quantité de la sortie cible pour une exécution de la certaine opération, le premier nombre d'exécution d'opérations étant un nombre d'exécution des certaines opérations à exécuter par premier moment unitaire, le second nombre d'exécution d'opérations étant un nombre d'exécution des certaines opérations à exécuter par un second moment unitaire différent du premier moment unitaire ;
**caractérisé en ce que** le processeur est en outre conçu pour :
exécuter (S3) un traitement fractionné pour chacun du premier nombre d'exécution d'opérations calculé et du second nombre d'exécution d'opérations calculé pour les changer en nombres entiers ; et
délivrer le nombre d'exécution d'opérations par moment unitaire de l'un du premier nombre d'exécution d'opérations et du second nombre d'exécution d'opérations ayant été changés en nombres entiers,
celui du premier nombre d'exécution d'opérations et du second nombre d'exécution d'opérations étant un nombre d'exécution d'opérations ayant une erreur d'arrondi plus petite (S6a) acquise avec le traitement fractionné, ou
celui du premier nombre d'exécution d'opérations et du second nombre d'exécution d'opérations étant un nombre d'exécution d'opérations ayant une erreur totale plus petite (S6) pour le moment unitaire requis généré avec une erreur d'arrondi acquise avec le traitement fractionné.

2. Dispositif de sortie (10) selon la revendication 1, **caractérisé en ce que**
la quantité totale de la sortie cible par une pluralité d'exécutions de la certaine opération exécutée par le dispositif externe est une quantité de transfusion totale de liquide de perfusion goutte à goutte à faire tomber,
le temps requis à passer pour une sortie de la cible de la quantité totale exécutée par le dispositif externe étant un temps requis à passer pour la chute du liquide de la perfusion goutte à goutte,
la quantité de la sortie cible pour une exécution de la certaine opération étant une quantité de liquide de la perfusion goutte à goutte par goutte,
le premier nombre d'exécution d'opérations étant un premier nombre de gouttes, et
le second nombre d'exécution d'opérations étant un second nombre de gouttes.

3. Dispositif de sortie (10) selon la revendication 2, **caractérisé en ce que** le processeur (11) notifie des chronométrages de goutte déterminés sur la base du nombre de gouttes de sortie par moment unitaire.

4. Dispositif de sortie (10) selon la revendication 3, **caractérisé en ce que** le processeur (11) notifie les chronométrages de goutte par affichage clignotant du nombre de gouttes de sortie par moment unitaire comme chronométrages de goutte.

5. Dispositif de sortie (10) selon l'une quelconque des revendications 1 à 4, **caractérisé en ce que** le second moment unitaire est plus long que le premier moment unitaire.

6. Procédé de sortie pour un dispositif de sortie (10), le procédé comprenant les étapes consistant à :
calculer (S2) un premier nombre d'exécution d'opérations et un second nombre d'exécution d'opérations, sur la base d'une quantité totale d'une sortie cible par une pluralité d'exécutions d'une certaine opération exécutée par un dispositif externe, d'un temps requis à passer pour une sortie de la cible de la quantité totale exécutée par le dispositif externe, et d'une quantité de la sortie cible pour une exécution de la certaine opération, le premier nombre d'exécution d'opérations étant un nombre d'exécution des certaines opérations à exécuter par un premier moment unitaire, le second nombre d'exécution d'opérations étant un nombre d'exécution des certaines opérations à exécuter par un second moment unitaire différent du premier moment unitaire ;
ledit procédé étant **caractérisé en ce qu'**il comprend en outre les étapes consistant à :
exécuter (S3) un traitement fractionné pour chacun du premier nombre d'exécution d'opérations calculé et du second nombre d'exécution d'opérations calculé pour les changer en nombres entiers ; et
délivrer le nombre d'exécution d'opérations par moment unitaire de l'un du premier nombre d'exécution d'opérations et du second nombre d'exécution d'opérations ayant été changés en nombres entiers,
celui du premier nombre d'exécution d'opérations et du second nombre d'exécution d'opérations étant un nombre d'exécution d'opérations ayant une erreur arrondie plus petite (S6a) acquise avec le traitement fractionné, ou
celui du premier nombre d'exécution d'opérations et du second nombre d'exécution d'opérations étant un nombre d'exécution d'opérations ayant une erreur totale plus petite (S6) pour le moment unitaire requis généré avec une erreur d'arrondi acquise avec le traitement fractionné.

7. Procédé de sortie selon la revendication 6, **caractérisé en ce que**
la quantité totale de la sortie cible par une pluralité d'exécutions de la certaine opération exécutée par le dispositif externe est une quantité de transfusion totale de liquide de perfusion goutte à goutte à faire tomber,
le temps requis à passer pour une sortie de la cible de la quantité totale exécutée par le dispositif externe étant un temps requis à passer pour la chute du liquide de la perfusion goutte à goutte,
la quantité de la sortie cible pour une exécution de la certaine opération étant une quantité de liquide de la perfusion goutte à goutte par goutte,
le premier nombre d'exécution d'opérations étant un premier nombre de gouttes, et
le second nombre d'exécution d'opérations étant un second nombre de gouttes.

8. Programme qui lorsqu'exécuté avec au moins un processeur (11) du dispositif de sortie (10) selon la revendication 1 exécute le procédé selon la revendication 6.

9. Programme selon la revendication 8, **caractérisé en ce que**
la quantité totale de la sortie cible par une pluralité d'exécutions de la certaine opération exécutée par le dispositif externe est une quantité de transfusion totale de liquide de perfusion goutte à goutte à faire tomber,
le temps requis à passer pour une sortie de la cible de la quantité totale exécutée par le dispositif externe étant un temps requis à passer pour la chute du liquide de la perfusion goutte à goutte, la quantité de la sortie cible pour une exécution de la certaine opération étant une quantité de liquide de la perfusion goutte à goutte par goutte,
le premier nombre d'exécution d'opérations étant un premier nombre de gouttes, et
le second nombre d'exécution d'opérations étant un second nombre de gouttes.
